# EUROPEAN PATENT APPLICATION

(11) **EP 3 181 849 A1**
(43) Date of publication of application: **21.06.2017**
(21) Application number: 16188803.7
(22) Date of filing: 14.09.2016
(51) Int. Cl.: F01N 11/00, F02D 41/12, F02D 41/14, F02D 41/22

(54) **A METHOD FOR TESTING A NOX SENSOR**

(30) Priority: 14.09.2015 US 201514852645
(71) Applicant: Deere & Company, Moline, IL 61265 (US)
(72) Inventor: BELL, Joseph A., Fairbank, IA Iowa 50629 (US); JOYNER, Taylor W., Waterloo, IA Iowa 50701 (US)
(74) Representative: Robson, Aidan John

(57) **Abstract**

A method for testing a sensor positioned downstream of an engine. The method includes motoring the engine, receiving a signal indicative of a value from the sensor, and determining whether the value is inside or outside of an established range. The established range is based on a known characteristic of an exhaust gas exiting from the engine as it is motoring.

## Description

### Field of the Disclosure

The present disclosure relates to a method for testing a NOₓ sensor.

### Background of the Disclosure

Diesel engines use a much leaner air-to-fuel ratio than gasoline engines. The larger amount of air in the intake gas promotes more complete fuel combustion and better fuel efficiency, and thus lower emissions of hydrocarbons and carbon monoxide than gasoline engines. However, with the higher pressures and temperatures in the diesel engine, nitrogen oxides emissions, which include nitrogen oxide ("NO") and nitrogen dioxide ("NO₂)", known collectively as "NOₓ," tend to be higher because the high temperatures cause the oxygen and nitrogen in the intake air to combine.

To comply with increasingly stringent government mandates regarding NOₓ emissions, engine manufacturers have developed several NOₓ reduction approaches. One such approach is exhaust gas recirculation ("EGR"), in which a percentage of the exhaust gas is drawn or forced back into the intake and mixed with the fresh intake gas and fuel that enters the combustion chamber. Another approach is selective catalytic reduction ("SCR"). The SCR process reduces NOₓ to diatomic nitrogen ("N₂") and water ("H₂O") using a catalyst and anhydrous ammonia ("NH₃") or aqueous NH₃, or a precursor that is convertible to NH₃, such as urea.

In addition to NOₓ emissions, diesel engines also produce particulate matter ("PM"), or soot, which is produced in comparatively larger amounts than that of gasoline engines. PM is a complex emission that includes elemental carbon, heavy hydrocarbons derived from the fuel, lubricating oil, and hydrated sulfuric acid derived from the fuel sulfur. One approach for reducing or removing PM in diesel exhaust is a diesel particle filter ("DPF"). The DPF is designed to collect PM while simultaneously allowing exhaust gases to pass therethrough.

One or more NOₓ sensors may be used in electronically-controlled diesel engines. The NOₓ sensor may fail, such that it provides an indication of NOₓ that is inaccurate. This false data supplied by the sensor may result in diagnostic trouble codes ("DTC") or performance complaints that do not directly implicate the failed sensor as the root cause. An example of such a performance complaint would be excessive diesel exhaust fluid ("DEF") consumption. Unlike a failed simple sensor, such as an engine coolant temperature sensor, it is difficult for a service technician to determine that the NOₓ signal is implausible through real-time observation of NOₓ, as measured by the sensor NOₓ measurement displayed in an electronic service tool, for example.

### Summary of the Disclosure

Disclosed is a method for testing a NOₓ sensor positioned downstream of an engine. The method includes motoring the engine, receiving a signal indicative of a value from the NOₓ sensor, and determining whether the value is inside or outside of an established range. The established range is based on a known characteristic of an exhaust gas exiting from the engine as it is motoring.

### Brief Description of the Drawings

The detailed description of the drawings refers to the accompanying figures in which:
FIG. 1 is a simplified schematic illustration of an example power system with a pair of NOₓ sensors; and
FIG. 2 is an example of a method for testing one or both of the NOₓ sensors.

### Detailed Description of the Drawings

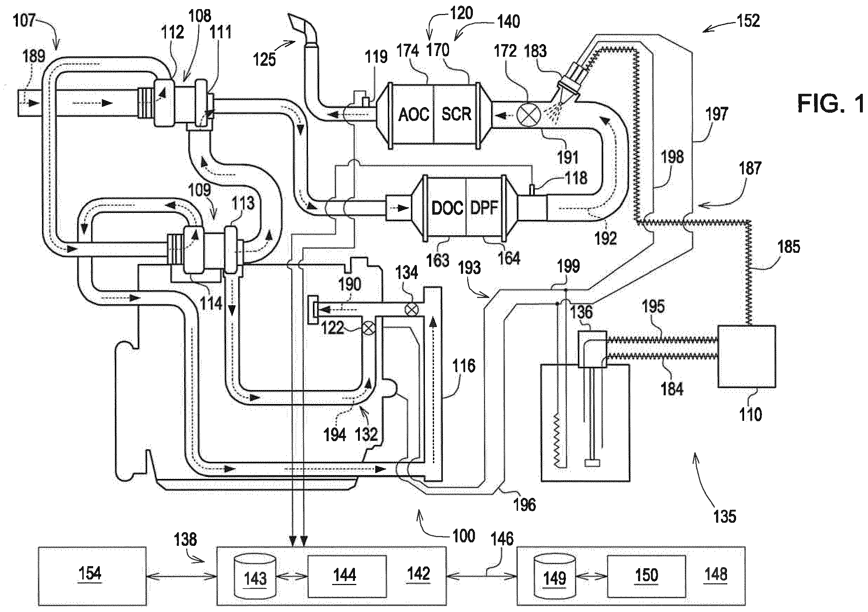

Referring to FIG 1, there is shown a schematic illustration of a power system 100 for providing power to a variety of machines, including on-highway trucks, construction vehicles, marine vessels, stationary generators, automobiles, agricultural vehicles, and recreational vehicles. The engine 106 may be any kind that produces an exhaust gas, as indicated by directional arrow 192. For example, engine 106 may be an internal combustion engine, such as a gasoline engine, a diesel engine, a gaseous fuel burning engine (e.g., natural gas), or any other exhaust gas producing engine. The engine 106 may be of any size, with any number cylinders, and in any configuration (e.g., "V," inline, and radial). The engine 106 may include various sensors, such as temperature sensors, pressure sensors, and mass flow sensors.

The power system 100 may include an intake system 107 that includes components for introducing a fresh intake gas, as indicated by directional arrow 189, into the engine 106. Among other things, the intake system 107 may include an intake manifold in communication with the cylinders, a compressor 112, a charge air cooler 116, and an air throttle actuator 134.

The compressor 112 may be a fixed geometry compressor, a variable geometry compressor, or any other type of compressor that is capable of receiving the fresh intake gas from upstream of the compressor 112. The compressor 112 compresses the fresh intake gas to an elevated pressure level. As shown, the charge air cooler 116 is positioned downstream of the compressor 112, and it cools the fresh intake gas.

Further, the power system 100 includes an exhaust system 140, which has components for directing exhaust gas from the engine 106 to the atmosphere. The pressure and volume of the exhaust gas drives the turbine 111, allowing it to drive the compressor 112 via a shaft. The combination of the compressor 112, the shaft, and the turbine 111 is known as a turbocharger 108.

Some embodiments of the power system 100 may also include a second turbocharger 109 that cooperates with the turbocharger 108 (i.e., series turbocharging). The second turbocharger 109 includes a second compressor 114, a second shaft, and a second turbine 113. The second compressor 114 may be a fixed geometry compressor, a variable geometry compressor, or any other type of compressor capable of receiving fresh intake gas, from upstream of the second compressor 114, and compressing the fresh intake gas to an elevated pressure level before it enters the engine 106.

The power system 100 may also have an EGR system 132 for receiving a recirculated portion of the exhaust gas, as indicated by directional arrow 194. The intake gas is indicated by directional arrow 190, and it is a combination of the fresh intake gas and the recirculated portion of the exhaust gas. The EGR system 132 may have an EGR valve 122 and an EGR mixer. The EGR valve 122 may allow a specific amount of the recirculated portion of the exhaust gas back into the intake manifold.

As further shown, the exhaust system 140 may include an aftertreatment system 120, and at least a portion of the exhaust gas passes therethrough. The aftertreatment system 120 removes various chemical compounds and particulate emissions present in the exhaust gas received from the engine 106.

The aftertreatment system 120 is shown having a diesel oxidation catalyst ("DOC") 163, a DPF 164, and an SCR system 152, though the need for such components depends on the particular size and application of the power system 100. The SCR system 152 has a reductant delivery system 135, an SCR catalyst 170, and an ammonia oxidation catalyst ("AOC") 174. The exhaust gas may flow through the DOC 163, the DPF 164, the SCR catalyst 170, and the AOC 174, and is then, as just mentioned, be expelled into the atmosphere. In other words, in the embodiment shown, the DPF 164 is positioned downstream of the DOC 163, the SCR catalyst 170 downstream of the DPF 164, and the AOC 174 downstream of the SCR catalyst 170. The DOC 163, the DPF 164, the SCR catalyst 170, and the AOC 174 may be coupled together. Exhaust gas that is treated in the aftertreatment system 120 and released into the atmosphere contains significantly fewer pollutants-such as PM, NOₓ, and hydrocarbons-than an untreated exhaust gas.

The DOC 163 may be configured in a variety of ways and contain catalyst materials useful in collecting, absorbing, and/or converting hydrocarbons, carbon monoxide, and/or oxides of nitrogen contained in the exhaust gas. Such catalyst materials may include, for example, aluminum, platinum, palladium, rhodium, barium, cerium, and/or alkali metals, alkaline-earth metals, rare-earth metals, or combinations thereof. The DOC 163 may include, for example, a ceramic substrate, a metallic mesh, foam, or any other porous material known in the art, and the catalyst materials may be located on, for example, a substrate of the DOC 163. The DOC(s) may also oxidize NO contained in the exhaust gas, thereby converting it to NO₂ upstream of the SCR catalyst 170.

The DPF 164 may be any of various particulate filters known in the art that are capable of reducing PM concentrations (e.g., soot and ash) in the exhaust gas, so as to meet requisite emission standards. Any structure capable of removing PM from the exhaust gas of the engine 106 may be used. For example, the DPF 164 may include a wall-flow ceramic substrate having a honeycomb cross-section constructed of cordierite, silicon carbide, or other suitable material to remove the PM. The DPF 164 may be electrically coupled to a controller, such as the ECU 142, that controls various characteristics of the DPF 164.

If the DPF 164 were used alone, it would initially help in meeting the emission requirements, but would quickly fill up with soot and need to be replaced. Therefore, the DPF 164 is combined with the DOC 163, which helps extend the life of the DPF 164 through the process of regeneration. The ECU 142 may measure the PM build up, also known as filter loading, in the DPF 164, using a combination of algorithms and sensors. When filter loading occurs, the ECU 142 manages the initiation and duration of the regeneration process.

Moreover, the reductant delivery system 135 may include a reductant tank 101 for storing the reductant. One example of a reductant is a solution having 32.5% high purity urea and 67.5% deionized water (e.g., DEF), which decomposes as it travels through a decomposition tube 191 to produce NH₃. Such a reductant may begin to freeze at approximately 12 deg F (-11 deg C). If the reductant freezes when a machine is shut down, then the reductant may need to be thawed before the SCR system 152 can function.

The reductant delivery system 135 may include a reductant header 136 mounted to the reductant tank 101, the reductant header 136 further including, in some embodiments, a level sensor 104 for measuring a quantity of the reductant in the reductant tank 101. The level sensor 104 may include a float configured to float at a liquid/air surface interface of reductant included within the reductant tank 101. Other implementations of the level sensor 104 are possible, and may include, for example, one or more of the following: (1) using one or more ultrasonic sensors, (2) using one or more optical liquid-surface measurement sensors, (3) using one or more pressure sensors disposed within the reductant tank 101, and (4) using one or more capacitance sensors.

In the illustrated embodiment, the reductant header 136 includes a tank heating element 105 that receives coolant from the engine 106. The power system 100 includes a cooling system 103 having a reductant coolant supply passage 187 and a reductant coolant return passage 193. The cooling system 103 may be an opened system or a closed system, depending on the specific application, while the coolant may be any form of engine coolant, including fresh water, sea water, an antifreeze mixture, and the like.

A first segment 196 of the reductant coolant supply passage 187 is positioned fluidly, between the engine 106 and the tank heating element 105, for supplying coolant to the tank heating element 130. The coolant circulates, through the tank heating element 130, so as to warm the reductant in the reductant tank 101, therefore reducing the risk that the reductant freezes therein and/or thawing the reductant upon startup. In an alternative embodiment, the tank heating element 105 may, instead, be an electrically resistive heating element. A second segment 197 of the reductant coolant supply passage 187 is positioned fluidly between the tank heating element 105 and a reductant delivery mechanism 183 for supplying coolant thereto. The coolant heats the reductant delivery mechanism 183, reducing the risk that reductant freezes therein.

A first segment 198 of the reductant coolant return passage 193 is positioned between the reductant delivery mechanism 183 and the tank heating element 130, and a second segment 199 of the reductant coolant return passage 193 is positioned between the engine 106 and the tank heating element 130. The first segment 198 and the second segment 199 return the coolant to the engine 106.

The decomposition tube 191 may be positioned downstream of the reductant delivery mechanism 183 but upstream of the SCR catalyst 170. The reductant delivery mechanism 183 may be, for example, an injector that is selectively controllable to inject reductant directly into the exhaust gas. As shown, the SCR system 152 may include a reductant mixer 172 that is positioned upstream of the SCR catalyst 170 and downstream of the reductant delivery mechanism 183.

The reductant delivery system 135 may additionally include a reductant pressure source and a reductant extraction passage 184. The reductant extraction passage 184 may be coupled fluidly to the reductant tank 101 and the reductant pressure source therebetween. Although the reductant extraction passage 184 is shown extending into the reductant tank 101, in other embodiments, the reductant extraction passage 184 may be coupled to an extraction tube via the reductant header 136. The reductant delivery system 135 may further include a reductant supply module 110, such as a Bosch reductant supply module (e.g., the Bosch Denoxtronic 2.2 - Urea Dosing System for SCR Systems).

The reductant delivery system 135 may also include a reductant dosing passage 185 and a reductant return passage 195. The reductant return passage 195 is shown extending into the reductant tank 101, though in some embodiments of the power system 100, the reductant return passage 195 may be coupled to a return tube via the reductant header 136. And the reductant delivery system 135 may have-among other things-valves, orifices, sensors, and pumps positioned in the reductant extraction passage 184, reductant dosing passage 185, and reductant return passage 195.

As mentioned above, one example of a reductant is a solution having 32.5% high purity urea and 67.5% deionized water (e.g., DEF), which decomposes as it travels through the decomposition tube 191 to produce NH₃. The NH₃ reacts with NOₓ in the presence of the SCR catalyst 170, and it reduces the NOₓ to less harmful emissions, such as N₂ and H₂O. The SCR catalyst 170 may be any of various catalysts known in the art. For example, in some embodiments, the SCR catalyst 170 may be a vanadium-based catalyst. But in other embodiments, the SCR catalyst 170 may be a zeolite-based catalyst, such as a Cu-zeolite or a Fe-zeolite. The AOC 174 may be any of various flowthrough catalysts for reacting with NH₃ and thereby produce nitrogen. Generally, the AOC 174 is utilized to remove NH₃ that has slipped through or exited the SCR catalyst 170. As shown, the AOC 174 and the SCR catalyst 170 may be positioned within the same housing, but in other embodiments, they may be separate from one another.

An electronic control system 138 of the engine 106 may include an electronic control unit ("ECU") 142 for monitoring and controlling the operation of the engine 106. As shown in FIG. 1, the ECU 142 may include a processor 144 and a memory 143 in communication with a processor 144. The processor 144 may be implemented using, for example, a microprocessor or other suitable processor. The memory 143 may be implemented using any suitable computer-readable media, and may include RAM and/or ROM.

The memory 143 may store software, such as algorithms and/or data, for configuring the processor 144 to perform one or more functions of the ECU 142. Alternatively, the ECU 142 may include discrete electronic circuits configured to perform such functions. In one embodiment, the ECU 142 may be operable to perform a torque estimation function. For example, the ECU 142 may be operable to estimate the average crankshaft torque produced by the engine 106. In another embodiment, the ECU 142 may be operable to perform a power loss detection function. For example, the ECU 142 may be operable to detect a power loss condition in one or more the cylinders of the engine 106. In another embodiment, the ECU 142 may be operable to perform a percent cylinder-power estimation function. For example, the ECU 142 may be operable to estimate the percentage of normal power achieved by one or more of the cylinders of the engine 106.

The ECU 142 may also include an input/output interface 146 for selectively communicating with a service tool 148, such as a diagnostic/service computer. The interface 146 may be implemented using any appropriate technology. For example, the interface 146 may be implemented using a wired or wireless data interface.

The service tool 148 may include a processor 150 and a memory 149 in communication therewith. The memory 149 may store software, such as algorithms and/or data, for configuring the processor 150 to perform one or more functions of the service tool 148. Alternatively, the service tool 148 may include discrete electronic circuits configured to perform such functions. The service tool 148 may also include an output device, such as a display screen and/or printer, for presenting output to an operator, and an input device, such as a keyboard and/or pointing device, for receiving commands and/or data from the operator.

The service tool 148 may be used to monitor and/or control the operation of the engine 106 and/or the electronic control system 138. For example, an operator (e.g., a service technician) may use the service tool 148 to perform diagnostic tests on the engine 106 and/or the electronic control system 138. The service tool 148 may also be used to program the processor 144 of the ECU 142. For example, an operator may use the service tool 148 to download new software into the memory 143 of the ECU 142 via interface 146. In an exemplary embodiment of the present disclosure, the service tool 148 may be used to calibrate functions performed by the ECU 142, as discussed below.

The electronic control system 138 may also include a display 154 in communication with the ECU 142. The display 154 may present information related to the operation of the engine 106 and/or the electronic control system 138. In one embodiment, the ECU 142 may control the display 154 to present data related to engine torque, power loss, and/or percent cylinder power. The display 154 may be implemented using any suitable type of display. For example, the display 154 may be implemented using a graphical and/or character display, such as a liquid crystal display ("LCD"). The display 154 may be located in a position visible to an operator of the engine 106. For example, the display 154 may be located in an operator station of a work machine powered by the engine 106.

The electronic control system 138 may also include one or more sensors for sensing operational parameters of the engine 106. For example, the system 138 may include a first NOₓ sensor 118 and a second NOₓ sensor 119, each of which senses a parameter indicative of a NOₓ content of the exhaust gas flowing thereby. The NOₓ sensors 118, 119 may, for example, rely upon an electrochemical or catalytic reaction that generates a current, the magnitude of which is indicative of the NOₓ concentration of the exhaust gas. The NOₓ sensors 118, 119 may be, for example, Continental "Smart NOₓ Sensors," and may measure O₂ levels in addition to measuring NOₓ levels. In the illustrated embodiment, the NOₓ sensor 118 is shown downstream of the DPF 164 but upstream of the SCR catalyst 170, while the NOₓ sensor 119 is shown downstream of the AOC 174. These are just two of the many possible locations for the NOₓ sensors 118, 119 in the aftertreatment system 120.

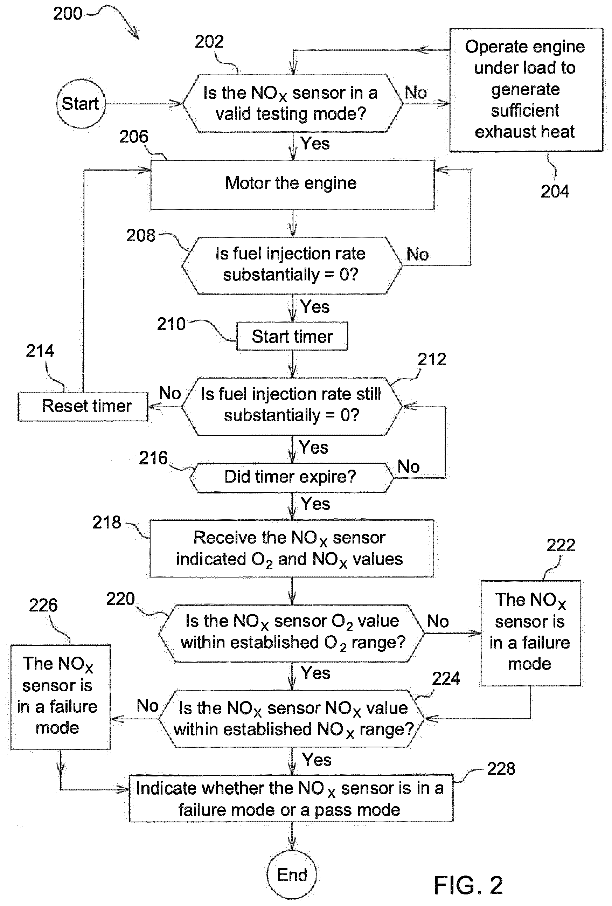

Referring to FIG. 2, there is shown an example of a method 200 for testing a NOₓ sensor, such as one or both of NOₓ sensors 118, 119. For simplicity, it is discussed with respect to only NOₓ sensor 118, but it could also or alternatively be discussed with respect to NOₓ sensor 119. The method 200 may be initiated by an operator of the vehicle, such as a driver or a service technician, and he may initiate it using the display 154 and/or using the service tool 148. In some embodiments, once initiated, the method 200 may proceed automatically without further aid from the operator.

The NOₓ sensor 118 is mounted to the aftertreatment system 120 during the test. This is to avoid removing the NOₓ sensor 118, which may damage it or the aftertreatment system 120 (e.g., damage to the threaded interface between the two). Further, the NOₓ sensor 118 may be prone to moisture damage, something that could occur when removed from the aftertreatment system 120 and tested in a laboratory environment. Additionally, even if the aftertreatment system 120 and NOₓ sensor 118 are not damaged, there are labor expenses associated with removing the NOₓ sensor 118, testing it in a lab, and then reinstalling it.

At step 202, the method 200 may determine whether the NOₓ sensor 118 is in a valid status mode. The valid status mode may be one that is at a sufficiently high temperature at the NOₓ sensor 118 (e.g., 500° F). If the NOₓ sensor 118 is not in the valid status mode, then the method 200 may proceed to step 204 and generate sufficiently high exhaust gas temperatures to raise the temperature of the NOₓ sensor 118 (e.g., increasing the speed or load of the engine 106).

At step 206, the method 200 may include motoring the engine 106, meaning that fresh intake gas flows through the engine 106, but no fuel is sprayed into the cylinders and combusted. The motoring may include, for example, driving the vehicle and then coasting it, or it may include, for example, driving a vehicle on a dyno. In either such example, the engine 106 will motor based on kinetic energy of the crank and transmission rotating, and will quickly slow down. While doing this, the engine 106 may initially be rotating at 2500 rpm, but then quickly decelerate down to 800 rpm, perhaps in only a few seconds.

As the engine 106 motors, fresh intake gas enters the intake system 107 and the engine 106, and then enters the aftertreatment system 120 as an exhaust gas. The fresh intake gas, which is simply just air from the atmosphere, has known physical characteristics, such as 0 ppm of NOₓ and 210,000 ppm of O₂. As the engine 106 motors, the fresh intake gas becomes the exhaust gas, and the exhaust gas has these same known physical characteristics. In some embodiments, the method 200 may command a heater associated with the NOₓ sensor 118 to turn on.

At step 208, the method 200 may determine whether a fuel injection rate is substantially equal to zero. Step 208 may act as a check for ensuring that the fuel injection rate is, in fact, equal to zero, and further ensuring that the exhaust gas will be transitioning to a chemical composition that is very similar, if not identical, to the fresh intake gas (i.e., air).

At step 210, the method 200 may start a timer if the fuel injection rate is substantially equal to zero. As the engine 106 is motoring and time is progressing, the exhaust gas will become progressively closer in chemical composition to the fresh intake gas. And further, as time progresses, even the recirculated exhaust gas would essentially be equivalent in chemical composition to the fresh intake gas.

At step 212, the method 200 may include confirming that the fuel injection quantity is still substantially equal to zero, so as to check one additional time. At step 214, the method 200 may include resetting the timer if the confirming indicates that the fuel injection quantity is not still substantially equal to zero. And then, at step 216, the method 200 may determine whether the time is expired.

At step 218, the method 200 may include receiving a signal indicative of a value from the NOₓ sensor 118. The signal may be indicative of at least one of a NOₓ value and an O₂ value associated with the exhaust gas flowing through the aftertreatment system 120.

At step 220, the method 200 may include determining whether the value is inside or outside of an established range, the established range being based on a known characteristic of an exhaust gas exiting from the engine 106 as it is motoring. Assuming that the signal is indicative of an O₂ value, then the method 200 may determine whether the O₂ value is inside of the established O₂ range, the established O₂ range being, for example, between 145,000 ppm and 230,000 ppm. This range may be broader or narrower (e.g., between 195,000 ppm and 215,000 ppm), depending on the specific embodiment of the method 200, but may be generally indicative of an exhaust gas that is similar in chemical composition to the fresh intake gas (i.e., air).

Comparing the NOₓ value to the established (and expected) NOₓ range may be a particularly useful test, particularly when the NOₓ sensor 118 is in an in-range failure mode. An in-range failure mode is one in which the NOₓ sensor 118 is providing a false signal that is within an acceptable range.

At step 220, the method 200 may determine that the NOₓ sensor 118 is in a pass mode-with respect to the O₂ value-if the O₂ value is inside of the established O₂ range. Alternatively, at steps 220 and 222, it may determine that the NOₓ sensor 118 is in a failure mode if the O₂ value is outside of the established O₂ range.

Assuming that the signal is also, or alternatively indicative of a NOₓ value, then the method 200 at step 224 may determine whether the value is inside or outside of an established NOₓ range, the established range being, for example, between 0 ppm and 300 ppm. This range may be broader or narrower (e.g., between 0 ppm and 75 ppm), depending on the specific embodiment of the method 200, but may generally be indicative of an exhaust gas that is similar in chemical composition to the fresh intake gas (i.e., air).

At step 224, the method 200 may determine that the NOₓ sensor 118 is in a pass mode-with respect to the NOₓ value-if the NOₓ value is inside of the established NOₓ range. Alternatively, at steps 224 and 226, it may determine that the NOₓ sensor 118 is in a failure mode if the NOₓ value is outside of the established NOₓ range.

At step 228, the method 200 may indicate a pass mode to an operator of the vehicle if the NOₓ value is inside of the established NOₓ range, and if the O₂ value is simultaneously inside of the established O₂ range. Or alternatively, at step 228, the method 200 may indicate a failure mode to an operator of the vehicle if at least one of the NOₓ value is outside of the established NOₓ range, and the O₂ value is outside of the established O₂ range. Some embodiments of the method 200 may only evaluate one of the NOₓ value and the O₂ value, instead of both.

The pass mode or failure mode may be indicated to the operator via the display 154 or the service tool, just to name a couple of examples. If the NOₓ sensor 118 is in the pass mode, then the operator knows the NOₓ sensor 118 is likely functioning correctly. If the NOₓ sensor 118 is in the failure mode, then the operator knows that the NOₓ sensor 118 is likely malfunctioning and needs to be replaced with a new sensor. Learning whether the NOₓ sensor 118 is in a pass mode of a failure mode, while still mounted to the aftertreatment system 120, is easier and more cost effective than performing a test of the NOₓ sensor 118 in a test lab after removing it from the aftertreatment system 120.

In an alternative embodiment of the method 200, the engine 106 may be off, and the residual exhaust gas has either dissipated or exited the aftertreatment system 120. This alternative is similar to the motoring condition, in that the gas that is in the aftertreatment system 120 is air, instead of a previously combusted exhaust gas that may have unknown quantities of O₂ and NOₓ, for example. The alternative embodiment may use many of the same steps as the illustrated example of the method 200.

While the disclosure has been illustrated and described in detail in the drawings and foregoing description, such illustration and description is to be considered as and not restrictive in character, it being understood that illustrative embodiments have been shown and described and that all changes and modifications that come within the spirit of the disclosure are desired to be protected. It will be noted that alternative embodiments of the present disclosure may not include all of the features described yet still benefit from at least some of the advantages of such features. Those of ordinary skill in the art may readily devise their own implementations that incorporate one or more of the features of the present disclosure and fall within the spirit and scope of the present invention as defined by the appended claims.

## Claims

1. A method for testing a NOₓ sensor positioned downstream of an engine, the method comprising:
motoring the engine;
receiving a signal indicative of a value from the NOₓ sensor; and
determining whether the value is inside or outside of an established range, the established range being based on a known characteristic of an exhaust gas exiting from the engine as it is motoring.

2. The method of claim 1, comprising generating a sufficient engine exhaust temperature if the NOₓ sensor is not in a valid testing mode.

3. The method of claim 1 or 2, wherein the engine is mounted to a vehicle, and the method comprises driving the vehicle, and the motoring comprises coasting the vehicle.

4. The method of claim 1, 2 or 3 comprising:
determining whether a fuel injection rate is equal to zero;
starting a timer if the fuel injection rate is equal to zero; and
determining whether the timer is expired.

5. The method of claim 1, 2, 3 or 4 wherein the signal is indicative of an O₂ value, the established range is an established O₂ range, and the determining comprises determining whether the O₂ value is inside of the established O₂ range.

6. The method of claim 5, wherein the established O₂ range is between 145,000 ppm and 230,000 ppm.

7. The method of claim 5, or 6 comprising determining that the NOₓ sensor is in a pass mode if the O₂ value is inside of the established O₂ range.

8. The method of claim 5, 6 or 7, wherein the engine is mounted to a vehicle, and the method comprises indicating the pass mode to an operator of the vehicle if the O₂ value is inside of the established O₂ range.

9. The method of claim 5, 6, 7 or 8 comprising determining that the NOₓ sensor is in a failure mode if the O₂ value is outside of the established O₂ range.

10. The method of claim 8 and 9, comprising indicating the failure mode to an operator of the vehicle if the O₂ value is outside of the established O₂ range.

11. The method of claim 1, wherein the signal is indicative of a NOₓ value, the established range is an established NOₓ range, and the determining comprises determining whether the NOₓ value is inside or outside of the established NOₓ range.

12. The method of claim 11, wherein the established NOₓ range is between 0 ppm and 300 ppm.

13. The method of claim 11 or 12, comprising determining that the NOₓ sensor is in a pass mode if the NOₓ value is inside of the established NOₓ range.

14. The method of claim 13, wherein the engine is mounted to a vehicle, and the method comprises indicating the pass mode to an operator of the vehicle if the NOₓ value is within the established NOₓ range.

15. The method of claim 11 or 12, comprising determining that the NOₓ sensor is in a failure mode if the NOₓ value is outside of the established NOₓ range.

16. The method of claim 15, wherein the engine is mounted to a vehicle, and the method comprises indicating the failure mode to an operator of the vehicle if the NOₓ value is outside of the established NOₓ range.

17. The method of claim 1, wherein the signal is indicative of a NOₓ value and an O₂ value, the established range comprises an established NOₓ range and an established O₂ range, and the determining comprises:
determining whether the NOₓ value is inside or outside of the established NOₓ range; and
determining whether the O₂ value is inside or outside of the established O₂ range.

18. The method of claim 17, wherein the established O₂ range is between 145,000 ppm and 230,000 ppm, and the established NOₓ range is between 0 ppm and 300 ppm.

19. The method of claim 17 or 18, wherein the engine is mounted to a vehicle, and the method comprises indicating a pass mode to an operator of the vehicle if the NOₓ value is inside of the established NOₓ range, and if the O₂ value is simultaneously inside of the established O₂ range.

20. The method of claim 17, wherein the engine is mounted to a vehicle, and the method comprises indicating a failure mode to an operator of the vehicle if at least one of the NOₓ value is outside of the established NOₓ range, and the O₂ value is outside of the established O₂ range.

21. A method for testing a NOₓ sensor positioned downstream of an engine, the method comprising:
receiving a signal indicative of a value from the NOₓ sensor; and
determining whether the value is inside or outside of an established range, the established range being based on a known characteristic of air.

22. The method of claim 21, wherein the signal is indicative of a NOₓ value and an O₂ value, the established range comprises an established NOₓ range and an established O₂ range, and the determining comprises:
determining whether the NOₓ value is inside or outside of the established NOₓ range; and
determining whether the O₂ value is inside or outside of the established O₂ range.

23. The method of claim 21 or 22, wherein the established O₂ range is between 145,000 ppm and 230,000 ppm, and the established NOₓ range is between 0 ppm and 300 ppm.

24. The method of claim 21 or 22, wherein the established O₂ range is between 195,000 ppm and 215,000 ppm, and the established NOₓ range is between 0 ppm and 75 ppm.
